# EUROPÄISCHE PATENTANMELDUNG

(11) **EP 2 143 406 A1**
(43) Veröffentlichungstag der Anmeldung: **13.01.2010**
(21) Anmeldenummer: 09162539.2
(22) Anmeldetag: 12.06.2009
(51) Int. Cl.: A61F 2/84

(54) **Einführsystem für eine medizinische Einrichtung mit einer Hülle**

(30) Priorität: 08.07.2008 DE 102008040252
(71) Anmelder: Biotronik VI Patent AG, 6341 Baar (CH)
(72) Erfinder: Hofmann, Eugen, 8049, Zürich (CH); Lootz, Daniel, 18119, Rostock (DE)
(74) Vertreter: Lindner-Vogt, Karin L.

(57) **Zusammenfassung**

Die Erfindung betrifft ein Einführsystem (100) für eine medizinische Einrichtung (10), die beim Einführen in einer Außenhülle (20) angeordnet ist, wobei die Außenhülle (20) einen Innenschaft wenigstens bereichsweise umgibt. Die Außenhülle (20) weist wenigstens auf ihrer der medizinischen Einrichtung (10) zugewandten Innenseite (29) wenigstens bereichsweise eine mit einer außenumfänglichen Segmentstruktur (12) der medizinischen Einrichtung (10) in der Art einer Schrauben-Muttern-Verbindung komplementäre Struktur (22, 32) auf.

## Beschreibung

Die Erfindung betrifft ein Einführsystem für eine medizinische Einrichtung mit einer Hülle sowie Hülle für ein Einführsystem für eine medizinische Einrichtung nach den Oberbegriffen der unabhängigen Ansprüche.

Zur Freisetzung von medizinischen Einrichtungen wie z.B. Stents oder Dilatationsballons kommen bekanntermaßen Katheter zum Einsatz. Ein Anwendungsgebiet betrifft z.B. die Angioplastie, bei der Ballonkatheter in Blutgefäße eingeführt, bis zu einer Stenose eingeschoben und dort aufgeweitet werden, um die Verengung des Blutgefäßes zu beseitigen. Üblicherweise ragt am distalen Ende ein Führungsdraht mit kleinem Durchmesser über den Ballonkatheter hinaus.

Das Einführen der medizinischen Einrichtung erfolgt mittels eines Einführsystems, einem so genannten Stent Delivery System. So ist aus der EP 1 601 312 B1 z.B. ein Einführsystem für einen Stent bekannt, bei dem der Stent zum besseren Positionieren des Stents auf einer drehbaren Hülle angeordnet ist. Speziell sollen damit beim Einführen des Stents Gabelungen im Blutgefäß besser passierbar gemacht werden.

Es gibt jedoch bestimmte Stentstrukturen, die mit den bekannten Einführsystemen, z.B. für selbstexpandierende Stents, nur schwer freigesetzt werden können. Die Freisetzung erfolgt, indem eine Außenhülle, die den Stent beim Einführen umschließt, zurückgezogen wird. Vor allem wenig vernetzte Stentstrukturen und insbesondere solche, deren Abstand zu Nachbarelementen groß ist, neigen dabei zum Aufstauchen und Blockieren, wenn sie frei gesetzt werden sollen. Ein Zurückziehen der Außenhülle wird erschwert oder sogar verhindert.

Der Erfindung liegt die Aufgabe zugrunde, ein Einführsystem für eine medizinische Einrichtung zu schaffen, bei der das Freisetzen der medizinischen Einrichtung erleichtert ist, auch wenn die Stentstruktur zum Aufstauchen neigt.

Es wird ein Einführsystem für eine medizinische Einrichtung vorgeschlagen, die beim Einführen in einer Außenhülle angeordnet ist, insbesondere ein Einführsystem für einen selbstexpandierenden Stent, wobei die Außenhülle einen Innenschaft wenigstens bereichsweise umgibt. Die Außenhülle weist wenigstens auf ihrer der medizinischen Einrichtung zugewandten Innenseite wenigstens bereichsweise eine mit einer außenumfänglichen Segmentstruktur der medizinischen Einrichtung in der Art einer Schrauben-Muttern-Verbindung komplementäre Struktur auf. Der Innenschaft trägt im distalen Bereich den Stent, während die Außenhülle den Stent in komprimiertem, gecrimpten Zustand hält. Vorteilhaft kann die Außenhülle rotierend zurückgezogen werden, selbst bei Stents, bei denen die überwiegende Anzahl der Stege der radialen Stützstruktur in einem Winkel zur axialen Richtung des Stents und somit der Außenhülle angeordnet sind.

Vorzugsweise kann beim Freisetzen der medizinischen Einrichtung die komplementäre Struktur der Außenhülle auf der Segmentstruktur in einer Drehbewegung zwangsführbar sein. Im Normalfall wird durch das Zurückziehen der Außenhülle der Stent freigesetzt. In diesem Fall kann die Außenhülle drehend zurückgezogen werden. Günstigerweise können damit axiale Stauchungskräfte, die dabei auf den Stent wirken, minimiert werden. Vorzugsweise kann die Drehbewegung der Außenhülle entlang einer spiralförmigen Meanderstruktur eines Stents verlaufen.

Vorzugsweise kann die Außenhülle torsionssteif ausgebildet sein, um eine Drehbewegung zu erleichtern.

Zweckmäßigerweise kann die Distanz, welche die Außenhülle pro Umdrehung in axialer Richtung bewegbar ist, auf eine Steigung der Segmentstruktur abgestimmt sein.

Kann die Außenhülle des Einführsystems wenigstens an ihrem proximalen Ende ein Außengewinde aufweisen, mit der eine Drehbewegung gegenüber der medizinischen Einrichtung aktivierbar ist, lässt sich die Freisetzung bewirken, indem z.B. eine Drehkurbel oder eine geeignete andere Vorrichtung betätigt wird, die auf das Außengewinde einwirken kann und die Außenhülle durch Drehen von der freizusetzenden medizinischen Einrichtung trennt.

Auch bei axial langen medizinischen Einrichtungen, z.B. Stents, kann eine derartige Freisetzung mit dem bevorzugten Einführsystem problemlos erfolgen, wenn die Außenhülle ein reibungsarmes Polymer umfassen kann. Bevorzugt sind z.B. Fluorpolymere wie etwa Teflon. Die Reibung zwischen der Segmentsstruktur der medizinischen Einrichtung und der Innenseite der Außenhülle kann vorteilhaft vermindert werden.

Bevorzugt kann die Außenhülle des Einführsystems auf der Innenseite als komplementäre Struktur zur Segmentstruktur der medizinischen Einrichtung eine Innengewindestruktur aufweisen.

Zur Erhöhung ihrer Torsionssteifigkeit kann die Außenhülle des Einführsystems günstigerweise eine Armierung aufweisen. So kann ein Geflecht aus einem oder mehreren Rund- oder Flachdrähten als Armierung auf einem Schlauch angeordnet sein. Die Armierung kann auch in den Schlauch eingelassen sein.

Die Außenhülle des Einführsystems kann ein Rohr umfassen, welches Schnitte mit einer Steigung aufweist. Die Schnitte dienen als Gewinde mit einer Steigung.

Vorteilhaft kann das Einführsystem eine Sperrvorrichtung aufweisen, um ein Verdrehen der medizinischen Einrichtung während des Freisetzens zu verhindern. So kann der Innenschaft mindestens im proximalen Bereich des Trägers der medizinischen Einrichtung so gestaltet sein, dass die medizinische Einrichtung fixiert ist. Auch kann zwischen dem proximalen und distalen Ende der Einführvorrichtung eine derartige Sperreinrichtung vorgesehen sein.

Die Außenhülle des Einführsystems kann wenigstens auf der Innenseite eine gewindeähnliche Struktur aufweisen, welche im Arbeitszustand das Ausführen einer Drehbewegung um eine komplementär ausgebildete Struktur ermöglicht.

Vorzugsweise kann die Struktur als Gleitspur oder Kerbe auf der Innenseite ausgebildet sein. Dabei kann die Gleitspur als Beschichtung, vorzugsweise als metallische Beschichtung, aufgetragen sein oder als gehärteter Bereich auf der Innenseite ausgebildet sein.

Eine solche Härtung kann durch eine geeignete Oberflächenbehandlung erfolgen, z.B. eine Härtung durch thermische Behandlung und/oder durch Bestrahlung mit Teilchen oder dergleichen.

Zur Verbesserung der Torsionssteifigkeit der Außenhülle kann zweckmäßigerweise eine Armierung vorgesehen sein.

Vorzugsweise kann an einem Ende der Außenhülle ein Außengewinde vorgesehen sein, mit dem die Drehbewegung der Außenhülle aktivierbar ist.

Die Außenhülle kann ein Rohr umfassen, welches Schnitte mit einer Steigung aufweist.

Es wird weiterhin eine Außenhülle für ein Einführsystem vorgeschlagen, bei der wenigstens auf der Innenseite eine gewindeähnliche Struktur ausgebildet ist, welche im Arbeitszustand das Ausführen einer Drehbewegung um eine komplementär ausgebildete Struktur ermöglicht.

Vorteilhaft kann die Struktur als Gleitspur oder Kerbe auf der Innenseite ausgebildet sein und/oder als Beschichtung aufgetragen sein. Die Gleitspur kann als gehärteter Bereich auf der Innenseite ausgebildet sein.

Zur Verbesserung der Torsionssteifigkeit kann zweckmäßigerweise eine Armierung vorgesehen ein, wenn der Körper der Außenhülle aus einem schlauch gebildet ist.

Die Außenhülle kann ein Rohr umfassen, welches Schnitte mit einer Steigung aufweist. Die Schnitte könne in ihrem Design mit Unterbrechungen, ihrer Steigung und ihrem Abstand so vorgesehen sein, dass die Torsionssteifigkeit der Außenhülle hinreichend gewährleistet ist.

Vorzugsweise kann an einem Ende der Außenhülle ein Außengewinde vorgesehen sein, mit dem die Drehbewegung der Außenhülle aktivierbar ist.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Die Erfindung ist nachfolgend beispielhaft anhand von in Zeichnungen dargestellten Ausführungsbeispielen näher erläutert. Es zeigen in schematischer Darstellung:
- Fig. 1: einen Schnitt durch eine bevorzugte Ausgestaltung eines Einführungssystems;
- Fig. 2: einen Stent mit einer stauchungsanfälligen Segmentstruktur.
- Fig. 3a, 3b: bevorzugte Ausgestaltungen von armierten Außenhüllen in Außenansicht;
- Fig. 4: eine Innenansicht einer Außenhülle mit Innengewinde;
- Fig. 5a, 5b: eine perspektivische Ansicht einer bevorzugten Ausgestaltung einer Außenhülle im Überblick (Fig. 5a) und als Detail (Fig. 5b); mit einem spiralförmigen Laserschnitt, der die Flexibilität des Rohres wesentlich erhöht. Je nach Steigung lässt sich die gewünschte Flexibilität einstellen.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typische Ausgestaltungen der Erfindung wieder und sollen die Erfindung nicht auf die dargestellten Ausgestaltungen beschränken.

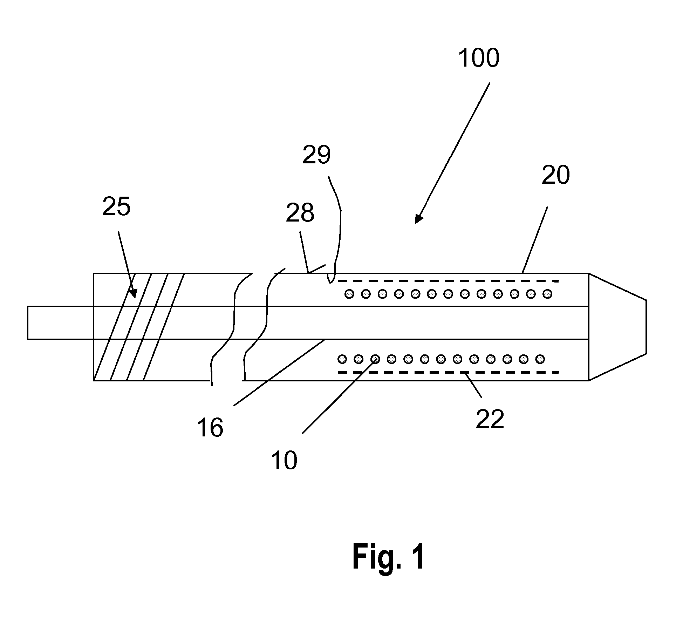

Fig. 1 zeigt schematisch ein bevorzugtes Einführsystem 100 für eine medizinische Einrichtung 10, die z.B. als selbstexpandierender Stent ausgebildet ist. Im montierten Zustand zum Einführen in ein Gefäß oder Organ ist die medizinische Einrichtung 10 auf dem distalen Ende eines Innenschafts 16 gelagert und von einer Außenhülle 20 umschlossen, die den Innenschaft 16 wenigstens bereichsweise umgibt. Zum Freisetzen des Stents wird die Außenhülle 20 zurückgezogen. Die Außenhülle 20 weist an ihrer Innenseite 29 eine Struktur 22 auf, die mit einer außenumfänglichen Struktur der medizinischen Einrichtung 10 so zusammenwirkt, dass beim Freisetzen eine Drehbewegung der Außenhülle 20 gegenüber der medizinischen Einrichtung 10 erfolgen kann, so dass eine axiale Stauchung vermieden werden kann.

Die Außenhülle 20 weist an ihrem proximalen Ende ein Außengewinde 25 auf, mit dem zum Freisetzen der medizinischen Einrichtung eine Drehbewegung aktiviert werden kann. Zweckmäßigerweise sind eine oder mehrere Sperrvorrichtungen vorgesehen (nicht dargestellt), mit denen ein unerwünschtes Verdrehen beim Einführen der medizinischen Einrichtung 10 an ihren Einsatzort unterbunden werden kann.

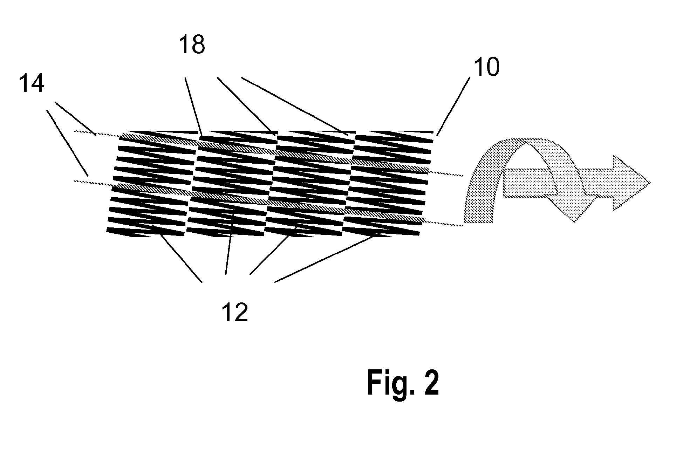

Die als Stent ausgebildete medizinische Einrichtung 10 kann eine Segmentstruktur 12 aufweisen, wie sie in Fig. 2 dargestellt ist. Die Segmentstruktur 12 besteht aus einer spiralförmig ausgerichteten Meanderstruktur (Strut-Elemente). Die Grenzen 18 der Segmentstrukturen 12 geben eine Steigung in der Art eines Gewindes vor. Wird die Außenhülle 20 axial, ohne Drehbewegung zurückgezogen, kann dies zu einer Aufstauchung der Segmentstrukturen führen. Gemäß einer bevorzugten Ausgestaltung der Erfindung kann die Außenhülle 20 wie eine Mutter auf einer Schraube gedreht werden, wobei die Drehbewegung auch eine axiale Verschiebung der Außenhülle 20 bewirkt. Diese Dreh-Hubbewegung ist durch die Pfeile rechts in der Fig. 2 angedeutet.

Die Außenhülle 20 (Fig. 1) weist vorzugsweise wenigstens auf ihrer der medizinischen Einrichtung 10 zugewandten Innenseite 29 wenigstens bereichsweise eine mit einer außenumfänglichen Segmentstruktur 12 der medizinischen Einrichtung 10 in der Art einer Schrauben-Muttern-Verbindung komplementäre Struktur 22 auf.

Durch die Steigung der Segmentstruktur 12 ist die Außenhülle 20 in einer Drehbewegung zwangsführbar. Die Distanz, um die die Außenhülle 20 pro Umdrehung in axialer Richtung bewegbar ist, ist dabei auf die Steigung der Segmentstruktur 12 abgestimmt.

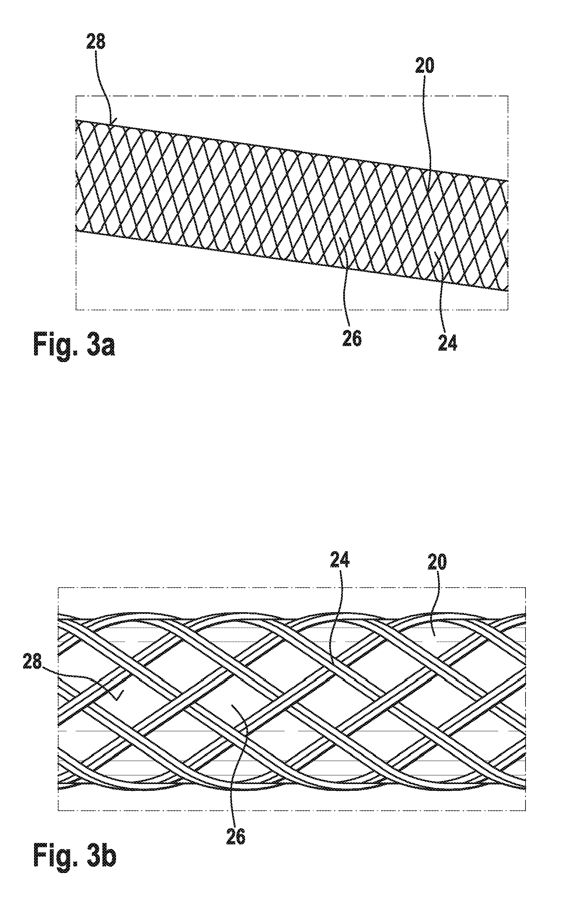

Die Außenhülle 20 ist zweckmäßigerweise torsionssteif ausgebildet. Die Fig. 3a und 3b zeigen Beispiele für eine torsionssteife Ausgestaltung einer Außenhülle 20. Der Körper 26 der Außenhülle 20 ist durch einen Schlauch gebildet, z.B. aus einem Polymer mit guten Gleiteigenschaften wie einem Fluorpolymer, um den ein Geflecht aus einem oder mehreren Rund- oder Flachdrähten als Armierung aufgebracht ist oder in den Schlauch eingelassen ist.

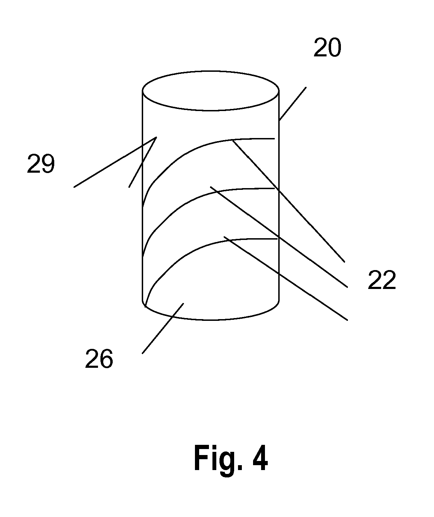

Fig. 4 zeigt beispielhaft eine Innenansicht einer Außenhülle 20 mit einem Polymerkörper 26, in den eine komplementäre Struktur 22 als Innengewinde eingearbeitet ist. Die komplementäre Struktur 22 kann als Gleitspur oder Kerbe auf der Innenseite 29 ausgebildet sein, die als z.B. metallische Beschichtung oder als gehärteter Bereich auf der Innenseite 29 ausgebildet sein kann.

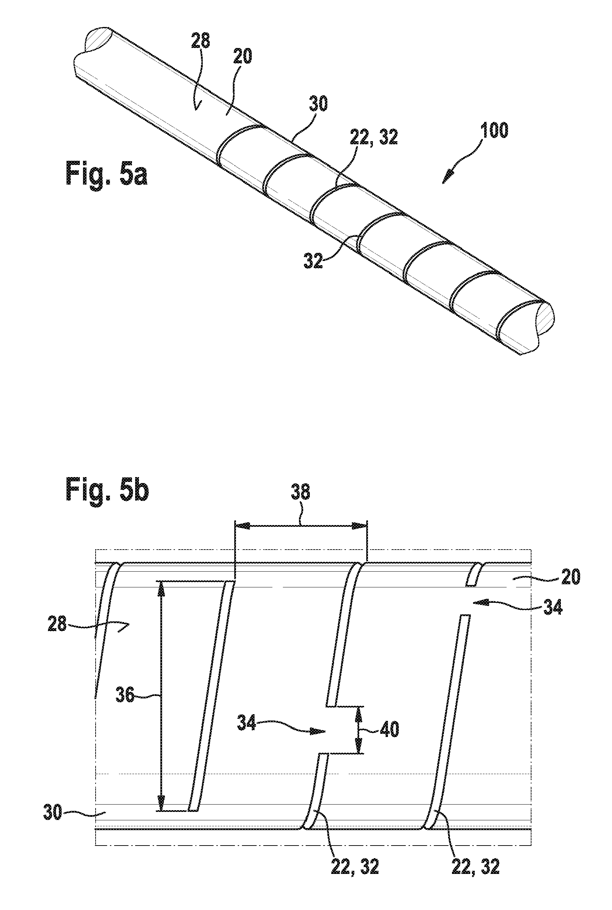

Fig. 5a und 5b zeigen eine bevorzugte alternative Ausgestaltung einer Außenhülle 20, die aus einem Körper 30 gebildet ist, in den als komplementäre Struktur 22 Schnitte 32 eingearbeitet sind. Durch die unterschiedlichen Ausführungen der Schnitte 32 in der Außenhülle 20 kann deren Steifigkeit beeinflusst werden. So kann die Länge 36 der Schnitte 32, deren axialer Abstand 38, sowie eine oder mehrere Unterbrechungen 40, ausgeführt durch ungeschnittene Teilstücke 34, gezielt so eingestellt werden, dass sich eine hinreichende Torsionssteifigkeit ergibt. Vorzugsweise ist die Außenhülle 20 aus Metall.

Es können mit dem erfindungsgemäßen Einführsystem 100 und der erfindungsgemäßen Außenhülle 20 vorteilhaft medizinische Einrichtungen 10, insbesondere Stentstrukturen, freigesetzt werden, die mit bekannten Einführsystemen nicht oder nur schwer frei zu setzen sind. Insbesondere kann eine Reduktion des Reibungswiderstandes und der damit verbundenen Freisetzkraft erreicht werden. Für die Konstruktion von Stentstrukturen erwächst der Vorteil, dass sie flexibler oder weniger empfindlich auf Ermüdungsbrüche konstruiert werden können.

## Patentansprüche

1. Einführsystem (100) für eine medizinische Einrichtung (10), die beim Einführen in einer Außenhülle (20) angeordnet ist, wobei die Außenhülle (20) einen Innenschaft wenigstens bereichsweise umgibt, **dadurch gekennzeichnet, dass** die Außenhülle (20) wenigstens auf ihrer der medizinischen Einrichtung (10) zugewandten Innenseite (29) wenigstens bereichsweise eine mit einer außenumfänglichen Segmentstruktur (12) der medizinischen Einrichtung (10) in der Art einer Schrauben-Muttern-Verbindung komplementäre Struktur (22, 32) aufweist.

2. Einführsystem nach Anspruch 1, **dadurch gekennzeichnet, dass** beim Freisetzen der medizinischen Einrichtung (10) die komplementäre Struktur (22, 32) der Außenhülle (20) auf der Segmentstruktur (12) in einer Drehbewegung zwangsführbar ist.

3. Einführsystem nach Anspruch 1 oder 2, **dadurch gekennzeichnet, dass** die Außenhülle (20) torsionssteif ausgebildet ist.

4. Einführsystem nach einem der vorhergehenden Ansprüche, **dadurch gekennzeichnet, dass** die Distanz, welche die Außenhülle (20) pro Umdrehung in axialer Richtung bewegbar ist, auf eine Steigung der Segmentstruktur (12) abgestimmt ist.

5. Einführsystem nach einem der vorhergehenden Ansprüche, **dadurch gekennzeichnet, dass** die Außenhülle (20) wenigstens an ihrem proximalen Ende ein Außengewinde aufweist, mit der eine Drehbewegung gegenüber der medizinischen Einrichtung (10) aktivierbar ist.

6. Einführsystem nach einem der vorhergehenden Ansprüche, **dadurch gekennzeichnet, dass** die Außenhülle (20) ein reibungsarmes Polymer umfasst.

7. Einführsystem nach einem der vorhergehenden Ansprüche, **dadurch gekennzeichnet, dass** die Außenhülle (20) auf der Innenseite (29) als komplementäre Struktur (22) eine Innengewindestruktur aufweist.

8. Einführsystem nach einem der vorhergehenden Ansprüche, **dadurch gekennzeichnet, dass** die Außenhülle (20) eine Armierung (24) zur Erhöhung ihrer Torsionssteifigkeit aufweist.

9. Einführsystem nach einem der vorhergehenden Ansprüche, **dadurch gekennzeichnet, dass** die Außenhülle (20) ein Rohr (30) umfasst, welches Schnitte (32) mit einer Steigung aufweist.

10. Außenhülle (20) für ein Einführsystem (100) nach Anspruch 1 oder 2, **dadurch gekennzeichnet, dass** wenigstens auf der Innenseite (29) eine gewindeähnliche Struktur (22, 32) ausgebildet ist, welche im Arbeitszustand das Ausführen einer Drehbewegung um einen komplementär ausgebildete Struktur (12) ermöglicht.

11. Außenhülle nach Anspruch 10, **dadurch gekennzeichnet, dass** die Struktur (22, 32) als Gleitspur oder Kerbe auf der Innenseite (29) ausgebildet ist.

12. Außenhülle nach Anspruch 11, **dadurch gekennzeichnet, dass** die Gleitspur als Beschichtung aufgetragen ist.

13. Außenhülle nach Anspruch 11 oder 12, **dadurch gekennzeichnet, dass** die Gleitspur als gehärteter Bereich auf der Innenseite (29) ausgebildet ist.

14. Außenhülle nach einem der Ansprüche 10 bis 13, **dadurch gekennzeichnet, dass** zur Verbesserung der Torsionssteifigkeit eine Armierung (24) vorgesehen ist.

15. Außenhülle nach einem der Ansprüche 10 bis 14, **dadurch gekennzeichnet, dass** an einem Ende ein Außengewinde vorgesehen ist.
